(19) Europäisches Patentamt
European Patent Office
Office européen des brevets

(11) **EP 4 684 798 A2**

(12) **EUROPEAN PATENT APPLICATION**

(43) Date of publication:
28.01.2026 Bulletin 2026/05

(51) International Patent Classification (IPC):
**A61K 39/395** (2006.01)

(21) Application number: 25212211.4

(22) Date of filing: **30.10.2025**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **12.09.2025 CN 202511307916**

(71) Applicant: **QILU PHARMACEUTICAL CO., LTD.**
**Jinan, Shandong 250100 (CN)**

(72) Inventors:
• **AN, Zhenming**
**Jinan (CN)**
• **LI, Daoyuan**
**Jinan (CN)**
• **GUO, Futeng**
**Jinan (CN)**
• **ZHANG, Xinqiang**
**Jinan (CN)**
• **YIN, Zhenhao**
**Jinan (CN)**
• **ZAN, Wenying**
**Jinan (CN)**
• **JI, Linyun**
**Jinan (CN)**
• **LIU, Chuanlei**
**Jinan (CN)**
• **WANG, Qingmin**
**Jinan (CN)**

(74) Representative: **Pons IP**
**Glorieta Rubén Darío 4**
**28010 Madrid (ES)**

Remarks:
The complete document including Reference Table(s) and the Sequence Listing(s) can be downloaded from the EPO website

(54) **A HIGH-CONCENTRATION COMPOSITION OF PD-1 ANTIBODY**

(57) Provided is a composition comprising Pembrolizumab and a variant thereof, wherein the variant is truncation variant with a N-terminal truncation of a heavy chain of Pembrolizumab, and comprises four peptide chains, wherein a first peptide chain comprises the amino acid sequence set forth in SEQ ID NO: 1 or SEQ ID NO: 3, a second peptide chain comprises the amino acid sequence set forth in SEQ ID NO: 3, and a third peptide chain and a fourth peptide chain comprise the amino acid sequence set forth in SEQ ID NO: 2, and wherein the content of the heavy chain comprising the N-terminal truncation is less than or equal to about 2%, expressed as a percentage of the content of the heavy chain comprising the N-terminal truncation accounting for the total content of heavy chains of Pembrolizumab and the variant thereof in the composition. Also provided is a pharmaceutical formulation comprising the composition.

EP 4 684 798 A2

**Description**

**CROSS-REFERENCE TO RELATED APPLICATION**

This application claims priority to Chinese Patent Application No.: CN 202511307916.6, filed on September 12, 2025, the entire contents of which are incorporated herein by reference for all purposes.

**TECHNICAL FIELD**

The present application relates to the field of pharmaceutical formulations. In particular, the present application provides a high-concentration composition of PD-1 antibody.

**BACKGROUND OF THE INVENTION**

With the continued development of therapeutic medicines of recombinant proteins, there is an increasingly urgent need to achieve subcutaneous administration of high-concentration medicines. In particular, in the field of treatment of chronic diseases such as tumors, there is an urgent need to develop antibody formulations with high concentration and small volume, in order to improve convenience of medication for patients and compliance of outpatients to the medication. Therefore, high-concentration dosage forms for subcutaneous injection have become one of the key development directions of differential research and development for therapeutic medicines of recombinant proteins.

Under normal physiological conditions, programmed death receptor 1 (PD-1) and programmed death ligand 1 (PD-L1) are important negative immunoregulatory factors that inhibit T cell activation and mediate the occurrence of infectious diseases and autoimmune diseases, and immune escape of tumor cells. However, during tumorigenesis, tumor cells abnormally express PD-L1, which transmits an inhibitory signal by binding to PD-1 on surface of T cells, thereby inhibiting activation and proliferation of the T cells, thus enabling the tumor cells to escape the surveillance and attack of the immune system and achieve the immune escape.

Pembrolizumab is a humanized monoclonal antibody that specifically binds to the PD-1 receptor on the surface of T cells and blocks the interaction between PD-1 and its ligands such as PD-L1, thereby relieving the immunosuppression of the tumor cells on the T cells, restoring the anti-tumor activity of the T cells, enhancing anti-tumor immune responses of the body, and achieving tumor cell killing.

Although high-concentration formulations have been developed for some antibody drugs, the production of high-concentration antibody formulations (e.g., at ≥100 mg/mL) is still confronted with significant challenges, including irreversible aggregation phenomena, irreversible precipitation and/or high viscosity issues, as well as the generation of potential new impurities. As a medication, Pembrolizumab must maintain its stability and efficacy. Quality control of pharmaceutical compositions mainly focuses on the control of content of active ingredient as well as contents of related substances (such as variants or impurities). In particular, the contents of related substances need to meet stringent pharmaceutical standards.

**SUMMARY OF THE INVENTION**

In a first aspect, the present application provides a composition comprising Pembrolizumab and a variant thereof, wherein the variant is a truncation variant with a N-terminal truncation of a heavy chain of Pembrolizumab, the variant comprising four peptide chains, wherein a first peptide chain of the variant comprises the amino acid sequence set forth in SEQ ID NO: 1 or SEQ ID NO: 3, a second peptide chain comprises the amino acid sequence set forth in SEQ ID NO: 3, and a third peptide chain and a fourth peptide chain comprise the amino acid sequence set forth in SEQ ID NO: 2, and wherein the content of the heavy chain comprising the N-terminal truncation is less than or equal to about 2%, expressed as a percentage of the content of the heavy chain comprising the N-terminal truncation accounting for the total content of heavy chains of Pembrolizumab and the variant thereof in the composition, which is calculated based on a trypsin-cleaved peptide mass fingerprinting analysis.

In a second aspect, the present application provides a pharmaceutical formulation comprising the composition of the first aspect, and one or more pharmaceutically acceptable carriers.

In a third aspect, the present application provides a method of detecting a Pembrolizumab variant in a Pembrolizumab-containing composition or pharmaceutical formulation, the variant being a truncation variant with a N-terminal truncation of a heavy chain of Pembrolizumab, the variant comprising four peptide chains, wherein a first peptide chain of the variant comprises the amino acid sequence set forth in SEQ ID NO: 1 or SEQ ID NO: 3, a second peptide chain comprises the amino acid sequence set forth in SEQ ID NO: 3, and a third peptide chain and a fourth peptide chain comprise the amino acid sequence set forth in SEQ ID NO: 2, the method comprising:

subjecting the composition or pharmaceutical formulation to enzymatic cleavage with trypsin to obtain an enzymatically cleaved product; and

subjecting the enzymatically cleaved product to a peptide mass fingerprinting analysis, e.g., a peptide mass fingerprinting analysis using LC-MS/MS, and determining the presence or absence of the Pembrolizumab variant based on the first-order and second-order mass spectra.

In a fourth aspect, the present application provides a method for quality inspection or quality control of a Pembrolizumab-containing product, comprising detecting the content of a Pembrolizumab variant in the Pembrolizumab-containing product, wherein the variant is a truncation variant with a N-terminal truncation of a heavy chain of Pembrolizumab, the variant comprising four peptide chains, wherein a first peptide chain of the variant comprises the amino acid sequence set forth in SEQ ID NO: 1 or SEQ ID NO: 3, a second peptide chain comprises the amino acid sequence set forth in SEQ ID NO: 3, and a third peptide chain and a fourth peptide chain comprise the amino acid sequence set forth in SEQ ID NO: 2.

In a fifth aspect, the present application provides use of a Pembrolizumab variant for quality inspection or quality control of a Pembrolizumab-containing product, the variant being a truncation variant with a N-terminal truncation of a heavy chain of Pembrolizumab, the variant comprising four peptide chains, wherein a first peptide chain of the variant comprises the amino acid sequence set forth in SEQ ID NO: 1 or SEQ ID NO: 3, a second peptide chain comprises the amino acid sequence set forth in SEQ ID NO: 3, and a third peptide chain and a fourth peptide chain comprise the amino acid sequence set forth in SEQ ID NO: 2.

In some embodiments, the composition, pharmaceutical formulation, or Pembrolizumab-containing product comprises at least 100 mg/ml, at least 110 mg/ml, at least 120 mg/ml, at least 130 mg/ml, at least 140 mg/ml, at least 150 mg/ml, at least 160 mg/ml, at least 170 mg/ml, at least 180 mg/ml, at least 190 mg/ml, or at least 200 mg/ml of Pembrolizumab and optionally the variant thereof.

**BRIEF DESCRIPTION OF THE DRAWINGS**

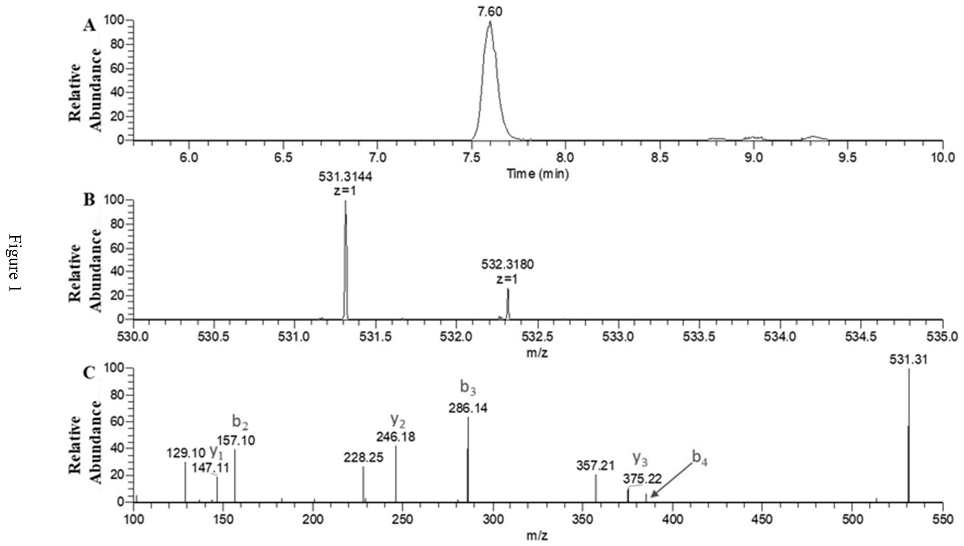

Figure 1 shows an extracted ion current chromatogram of a first-order mass spectrum (Panel A), a first-order mass spectrum (Panel B), and a second-order mass spectrum (Panel C) of a truncated peptide segment of exemplary truncation variant S7 in a Pembrolizumab sample.

**DESCRIPTION OF SEQUENCES**

SEQ ID NO:1 is the amino acid sequence of two identical heavy chains of Pembrolizumab, and is shown as below (note that the underlined portions are CDR domains annotated according to the Kabat numbering scheme):

QVQLVQSGVEVKKPGASVKVSCKASGYTFT<u>NYYMY</u>WVRQAPGQGLEW

MG<u>GINPSNGGTNFNEKFKN</u>RVTLTTDSSTTTAYMELKSLQFDDTAVYYCAR<u>R</u>

<u>DYRFDMGFDY</u>WGQGTTVTVSSASTKGPSVFPLAPCSRSTSESTAALGCLVKD

YFPEPVTVSWNSGALTSGVHTFPAVLQSSGLYSLSSVVTVPSSSLGTKTYTCN

VDHKPSNTKVDKRVESKYGPPCPPCPAPEFLGGPSVFLFPPKPKDTLMISRTPE

VTCVVVDVSQEDPEVQFNWYVDGVEVHNAKTKPREEQFNSTYRVVSVLTVL

HQDWLNGKEYKCKVSNKGLPSSIEKTISKAKGQPREPQVYTLPPSQEEMTKN

QVSLTCLVKGFYPSDIAVEWESNGQPENNYKTTPPVLDSDGSFFLYSRLTVDK

SRWQEGNVFSCSVMHEALHNHYTQKSLSLSLGK

SEQ ID NO:2 is the amino acid sequence of two identical light chains of Pembrolizumab, and is shown as below (note that the underlined portions are CDR domains annotated according to the Kabat numbering scheme):

EIVLTQSPATLSLSPGERATLSCRASKGVSTSGYSYLHWYQQKPGQAPRL

LIYLASYLESGVPARFSGSGSGTDFTLTISSLEPEDFAVYYCQHSRDLPLTFGG

GTKVEIKRTVAAPSVFIFPPSDEQLKSGTASVVCLLNNFYPREAKVQWKVDN

ALQSGNSQESVTEQDSKDSTYSLSSTLTLSKADYEKHKVYACEVTHQGLSSP

VTKSFNRGEC

SEQ ID NO:3 is the amino acid sequence of the heavy chain comprising the N-terminal truncation in the truncation variant of Pembrolizumab (one heavy chain of the truncation variant comprises the N-terminal truncation at the serine at position 7, as compared to Pembrolizumab) (note that the underlined portions are CDR domains annotated according to the Kabat numbering scheme):

GVEVKKPGASVKVSCKASGYTFTNYYMYWVRQAPGQGLEWMGGINPS

NGGTNFNEKFKNRVTLTTDSSTTTAYMELKSLQFDDTAVYYCARRDYRFDM

GFDYWGQGTTVTVSSASTKGPSVFPLAPCSRSTSESTAALGCLVKDYFPEPVT

VSWNSGALTSGVHTFPAVLQSSGLYSLSSVVTVPSSSLGTKTYTCNVDHKPSN

TKVDKRVESKYGPPCPPCPAPEFLGGPSVFLFPPKPKDTLMISRTPEVTCVVVD

VSQEDPEVQFNWYVDGVEVHNAKTKPREEQFNSTYRVVSVLTVLHQDWLN

GKEYKCKVSNKGLPSSIEKTISKAKGQPREPQVYTLPPSQEEMTKNQVSLTCL

VKGFYPSDIAVEWESNGQPENNYKTTPPVLDSDGSFFLYSRLTVDKSRWQEG

NVFSCSVMHEALHNHYTQKSLSLSLGK

SEQ ID NO:4 is the amino acid sequence of the longer heavy chain in the sequence of S7 for recombinant expression, and is shown as below (note that the residues shown in italics are mutated amino acids):

QVQLVQSGVEVKKPGASVKVSCKASGYTFTNYYMYWVRQAPGQGLEW

MGGINPSNGGTNFNEKFKNRVTLTTDSSTTTAYMELKSLQFDDTAVYYCARR

DYRFDMGFDYWGQGTTVTVSSASTKGPSVFPLAPCSRSTSESTAALGCLVKD

YFPEPVTVSWNSGALTSGVHTFPAVLQSSGLYSLSSVVTVPSSSLGTKTYTCNV

DHKPSNTKVDKRVESKYGPPCPPCPAPEFLGGPSVFLFPPKPKDTLMISRTPEV

TCVVVDVSQEDPEVQFNWYVDGVEVHNAKTKPREEQFNSTYRVVSVLTVLH

QDWLNGKEYKCKVSNKGLPSSIEKTISKAKGQPREPQV*C*TLPPSQEEMTKNQ

VSL*SCA*VKGFYPSDIAVEWESNGQPENNYKTTPPVLDSDGSFFL*V*SRLTVDKSR

WQEGNVFSCSVMHEALHN*RF*TQKSLSLSLGK

SEQ ID NO:5 is the amino acid sequence of the shorter heavy chain in the sequence S7 for recombinant expression, and is shown as below (note that the residues shown in italics are mutated amino acids):

GVEVKKPGASVKVSCKASGYTFTNYYMYWVRQAPGQGLEWMGGINPS

NGGTNFNEKFKNRVTLTTDSSTTTAYMELKSLQFDDTAVYYCARRDYRFDMG

FDYWGQGTTVTVSSASTKGPSVFPLAPCSRSTSESTAALGCLVKDYFPEPVTV

SWNSGALTSGVHTFPAVLQSSGLYSLSSVVTVPSSSLGTKTYTCNVDHKPSNT

KVDKRVESKYGPPCPPCPAPEFLGGPSVFLFPPKPKDTLMISRTPEVTCVVVDV

SQEDPEVQFNWYVDGVEVHNAKTKPREEQFNSTYRVVSVLTVLHQDWLNGK

EYKCKVSNKGLPSSIEKTISKAKGQPREPQVYTLPP*C*QEEMTKNQVSL*W*CLVK

GFYPSDIAVEWESNGQPENNYKTTPPVLDSDGSFFLYSRLTVDKSRWQEGNVF

SCSVMHEALHNHYTQKSLSLSLGK

**DETAILED DESCRIPTION**

In the specification and the claims, the term "Pembrolizumab" refers to a monoclonal antibody formed from two identical heavy chains and two identical light chains joined by disulfide bonds, wherein the sequence of the two identical heavy chains is set forth in SEQ ID NO: 1, while the sequence of the two identical light chains is set forth in SEQ ID NO:2.

In the specification and the claims, the term "about" refers to a range of values considered by one of ordinary skill in the art to be equivalent to the recited value (e.g., having the same function or result), e.g., +/-10% of the recited value.

In the specification and the claims, the terms "including", "comprising" and "containing" mean "comprising but not limited to", and do not intend to exclude other parts, additives, components or steps.

The inventors of the present application have surprisingly found that Pembrolizumab comprising a truncation, e.g., a Pembrolizumab variant comprising an N-terminal truncation, e.g., the N-terminal truncation being located at the serine at position 7 of at least one heavy chain of Pembrolizumab, was produced during the preparation of Pembrolizumab.

In some embodiments, the content of the heavy chain comprising the N-terminal truncation of Pembrolizumab is less than or equal to about 2% of the total content of heavy chains of Pembrolizumab in the composition, which does not affect the binding activity and/or thermostability of the Pembrolizumab sample. Thus, in these embodiments, if the content of the heavy chain comprising the N-terminal truncation of Pembrolizumab is evaluated to be no more than about 2% during the preparation of Pembrolizumab, there is no need to perform operations of removing variants or impurities in general, which simplifies the production process and saves production costs to some extent.

In a first aspect, the present application provides a composition comprising Pembrolizumab and a variant thereof, wherein the variant is a truncation variant with a N-terminal truncation of a heavy chain of Pembrolizumab, the variant comprising four peptide chains, wherein a first peptide chain of the variant comprises the amino acid sequence set forth in SEQ ID NO: 1 or SEQ ID NO: 3, a second peptide chain comprises the amino acid sequence set forth in SEQ ID NO: 3, and a third peptide chain and a fourth peptide chain comprise the amino acid sequence set forth in SEQ ID NO: 2.

In some embodiments of the first aspect, the composition comprises at least 100 mg/ml, at least 110 mg/ml, at least 120 mg/ml, at least 130 mg/ml, at least 140 mg/ml, at least 150 mg/ml, at least 160 mg/ml, at least 170 mg/ml, at least 180 mg/ml, at least 190 mg/ml, or at least 200 mg/ml of Pembrolizumab and/or the variant thereof.

In some embodiments of the first aspect, the content of the heavy chain comprising the N-terminal truncation is less than or equal to about 2%, expressed as a percentage of the content of the heavy chain comprising the N-terminal truncation accounting for the total content of heavy chains of Pembrolizumab and the variant thereof in the composition, which is calculated based on a trypsin-cleaved peptide mass fingerprinting analysis.

In some embodiments of the first aspect, the content of the heavy chain comprising the N-terminal truncation is greater than or equal to about 0.001%, expressed as a percentage of the content of the heavy chain comprising the N-terminal truncation accounting for the total content of heavy chains of Pembrolizumab and the variant thereof in the composition, which is calculated based on a trypsin-cleaved peptide mass fingerprinting analysis.

In some embodiments of the first aspect, the content of the heavy chain comprising the N-terminal truncation is less than or equal to about 2.0%, less than or equal to about 1.9%, less than or equal to about 1.8%, less than or equal to about 1.7%, less than or equal to about 1.6%, less than or equal to about 1.5%, less than or equal to about 1.4%, less than or equal to about 1.3%, less than or equal to about 1.2%, less than or equal to about 1.1%, less than or equal to about 1.0%, less than or equal to about 0.9%, less than or equal to about 0.8%, less than or equal to about 0.7%, less than or equal to about 0.6%, less than or equal to about 0.5%, less than or equal to about 0.4%, less than or equal to about 0.3%, less than or equal to about 0.2%, less than or equal to about 0.1%, less than or equal to about 0.05%, or less than or equal to about 0.01%, expressed as a percentage of the content of the heavy chain comprising the N-terminal truncation accounting for the total content of heavy chains of Pembrolizumab and the variant thereof in the composition, which is calculated based on a trypsin-cleaved peptide mass fingerprinting analysis.

In some embodiments of the first aspect, the content of the heavy chain comprising the N-terminal truncation is from 0.001% to 2.0%, or any interval within any of the foregoing ranges or any value within the interval, expressed as a percentage of the content of the heavy chain comprising the N-terminal truncation accounting for the total content of heavy chains of Pembrolizumab and the variant thereof in the composition, which is calculated based on a trypsin-cleaved peptide mass fingerprinting analysis.

In some embodiments of the first aspect, the content of the heavy chain comprising the N-terminal truncation is 0.001%, 0.005%, 0.01%, 0.05%, 0.1%, 0.2%, 0.4%, 0.6%, 0.8%, 1.0%, 1.1%, 1.2%, 1.3%, 1.4%, 1.5%, 1.6%, 1.7%, 1.8%, 1.9%, 2.0%, or any interval within the preceding values or ranges, or any value within the interval, expressed as a percentage of the content of the heavy chain comprising the N-terminal truncation accounting for the total content of heavy chains of Pembrolizumab and the variant thereof in the composition, which is calculated based on a trypsin-cleaved peptide mass fingerprinting analysis.

It is to be understood that although the above reference to the content of the Pembrolizumab variant comprising the N-terminal truncation is expressed as a percentage of the content of the heavy chain comprising the N-terminal truncation accounting for the total content of all heavy chains in the composition, this is only one way of expression. It will be appreciated by those skilled in the art that the content of the Pembrolizumab variant comprising the N-terminal truncation may also be expressed in a variety of other ways, such as by the percentage of the content of a peptide segment comprising the N-terminal truncation (which may be referred to as a "truncated peptide segment") accounting for the sum of the contents of the truncated peptide segment and the corresponding untruncated peptide segment (e.g., having the same or substantially the same amino acid sequence but excluding the N-terminal truncation) in the composition.

Those skilled in the art may measure and determine the content of the Pembrolizumab variant comprising the N-terminal truncation in the composition in a variety of ways. For example, in some embodiments, the content may be measured and determined by the following method:

(a) adding urea at a final concentration of 6 M and 10 mM of TCEP to a certain amount of a sample, performing incubation at 37°C for 20 min for denaturation and reduction, and then adding IAM at a final concentration of 20 mM and performing incubation at 37°C for 15 min for alkylation;

(b) then adding a solution of 50 mM ammonium bicarbonate to dilute urea to a concentration of 1 M, followed by adding sequencing-grade trypsin at a ratio of protein: enzyme = 10:1 (mass ratio) to perform enzymatic cleavage at 37°C for 25 min, and at the end of the enzymatic cleavage, adding formic acid at a final concentration of 1% to stop the enzymatic cleavage; and

(c) subjecting the sample to centrifugation followed by a peptide mass fingerprint analysis with liquid chromatography-tandem high-resolution mass spectrometry, in which peptide segments are separated with a C18 column and then analyzed with the first-order mass spectrometry and second-order mass spectrometry. Then, XICs of the truncated peptide segment (GVEVK (SEQ ID NO: 6); the mass-to-charge ratios of main ions are 531.31 and 266.16, respectively) and its corresponding untruncated peptide segment (QVQLVQSGVEVK (SEQ ID NO: 7); the mass-to-charge ratios of main ions are 657.36, 438.59, 1296.74 and 648.87, respectively) are extracted respectively, and the mass deviation is set to 20 ppm. Peak areas of the respective target components are obtained by integration, and the truncation ratio is calculated according to the following formula:

$$\frac{\text{content of}}{\text{N-terminal truncation-containing variant}} = \frac{\text{XIC peak area of truncated peptide segment}}{\text{XIC peak area of truncated peptide segment + XIC peak area of untruncated peptide segment}} \times 100\%$$

In a second aspect, the present application provides a pharmaceutical formulation comprising the composition of the first aspect, and one or more pharmaceutically acceptable carriers.

The pharmaceutically acceptable carrier is non-toxic to a recipient at the dosages and concentrations employed. In some embodiments, the pharmaceutically acceptable carrier includes, for example, water; a buffer, such as phosphate, citrate and another organic acid; an antioxidant, such as ascorbic acid and methionine; a preservative; a hydrophilic polymer, such as polyvinylpyrrolidone; a chelating agent, such as EDTA, and the like.

In some embodiments, the pharmaceutical formulation may further comprise one or more of the following: a lubricant, such as talc, magnesium stearate, and mineral oil; a wetting agent; an emulsifier; a suspending agent; a preservative, such as benzoic acid, sorbic acid and calcium propionate; a sweetening agent and/or a flavoring agent, and the like.

In some embodiments, the composition, pharmaceutical formulation, and/or Pembrolizumab-containing product in the present application may comprise one or more of histidine, sucrose, L-arginine, methionine, polysorbate 80, and hyaluronidase.

In some embodiments, the composition, pharmaceutical formulation, and/or Pembrolizumab-containing product in the present application may be formulated in a form of a tablet, a pill, a powder, a lozenge, an elixir, a suspension, an emulsion, a solution, a syrup, a suppository, a capsule, or the like.

In some embodiments, the composition, pharmaceutical formulation, and/or Pembrolizumab-containing product in the present application may be delivered with any physiologically acceptable administration mode which includes, but is not limited to, oral administration, parenteral administration, nasal administration, rectal administration, intraperitoneal administration, intravascular injection, subcutaneous administration, transdermal administration, inhalation administration, and the like.

In some embodiments, the pharmaceutical formulation for *in vivo* administration must be sterilized. This can be easily achieved by using a sterile filter membrane for filtration.

In some embodiments, the pharmaceutical formulation for therapeutic use may be formulated in the form of a lyophilized formulation or aqueous solution for storage by mixing a reagent of the desired purity with a pharmaceutically acceptable carrier, an excipient, and the like, as appropriate.

In a third aspect, the present application provides a method of detecting a Pembrolizumab variant in a Pembrolizumab-containing composition or pharmaceutical formulation, wherein the variant is a truncation variant with a N-terminal truncation of a heavy chain of Pembrolizumab, the variant comprising four peptide chains, wherein a first peptide chain of the variant comprises the amino acid sequence set forth in SEQ ID NO: 1 or SEQ ID NO: 3, a second peptide chain comprises the amino acid sequence set forth in SEQ ID NO: 3, and a third peptide chain and a fourth peptide chain comprise the amino acid sequence set forth in SEQ ID NO: 2, the method comprising:

subjecting the composition or pharmaceutical formulation to enzymatic cleavage with trypsin to obtain an enzymatically cleaved product; and

subjecting the enzymatically cleaved product to a peptide mass fingerprinting analysis, e.g., a peptide mass fingerprinting analysis using LC-MS/MS, and determining the presence or absence of Pembrolizumab variant based on the first-order and second-order mass spectra.

In some embodiments of the third aspect, the content of Pembrolizumab or the variant thereof in the Pembrolizumab-containing composition or pharmaceutical formulation is as described in the first aspect.

In some embodiments of the third aspect, the method further comprises obtaining, after the peptide mass fingerprint analysis, extracted ion current chromatograms (XICs) of a peptide segment comprising the N-terminal truncation of the heavy chain and an untruncated peptide segment, and calculating the content of the Pembrolizumab variant comprising the N-terminal truncation of the heavy chain based on the corresponding peak areas.

In a fourth aspect, the present application provides a method for quality inspection or quality control of a Pembrolizumab-containing product, comprising detecting the content of a Pembrolizumab variant in the Pembrolizumab-containing product, wherein the variant is a truncation variant with a N-terminal truncation of a heavy chain of Pembrolizumab, the variant comprising four peptide chains, wherein a first peptide chain of the variant comprises the amino acid sequence set forth in SEQ ID NO: 1 or SEQ ID NO: 3, a second peptide chain comprises the amino acid sequence set forth in SEQ ID NO: 3, and a third peptide chain and a fourth peptide chain comprise the amino acid sequence set forth in SEQ ID NO: 2.

In some embodiments, the content of the heavy chain comprising the N-terminal truncation is less than or equal to about 2.0%, less than or equal to about 1.9%, less than or equal to about 1.8%, less than or equal to about 1.7%, less than or equal to about 1.6%, less than or equal to about 1.5%, less than or equal to about 1.4%, less than or equal to about 1.3%, less than or equal to about 1.2%, less than or equal to about 1.1%, less than or equal to about 1.0%, less than or equal to about 0.9%, less than or equal to about 0.8%, less than or equal to about 0.7%, less than or equal to about 0.6%, less than or equal to about 0.5%, less than or equal to about 0.4%, less than or equal to about 0.3%, less than or equal to about 0.2%, less than or equal to about 0.1%, less than or equal to about 0.05%, or less than or equal to about 0.01%, expressed as a percentage of the content of the heavy chain comprising the N-terminal truncation accounting for the total content of heavy chains of Pembrolizumab and the variant thereof in the composition, indicating that the product meets pharmaceutical requirements.

In a fifth aspect, the present application provides use of a Pembrolizumab variant for quality inspection or quality control of a Pembrolizumab-containing product, wherein the variant is a truncation variant with a N-terminal truncation of a heavy chain of Pembrolizumab, the variant comprising four peptide chains, wherein a first peptide chain of the variant comprises the amino acid sequence set forth in SEQ ID NO: 1 or SEQ ID NO: 3, a second peptide chain comprises the amino acid sequence set forth in SEQ ID NO: 3, and a third peptide chain and a fourth peptide chain comprise the amino acid sequence set forth in SEQ ID NO: 2.

In some embodiments of the fourth aspect and/or the fifth aspect, the content of Pembrolizumab or the Pembrolizumab variant comprising the heavy chain N-terminal truncation in the product is as described in the first aspect.

In some embodiments of the present application, the Pembrolizumab variant comprises four peptide chains, wherein the first peptide chain of the variant comprises the amino acid sequence set forth in SEQ ID NO: 1, the second peptide chain comprises the amino acid sequence set forth in SEQ ID NO: 3, and the third peptide chain and the fourth peptide chain comprise the amino acid sequence set forth in SEQ ID NO: 2.

In some embodiments of the present application, the Pembrolizumab variant comprises four peptide chains, wherein the first peptide chain of the variant comprises the amino acid sequence set forth in SEQ ID NO: 3, the second peptide chain comprises the amino acid sequence set forth in SEQ ID NO: 3, and the third peptide chain and the fourth peptide chain comprise the amino acid sequence set forth in SEQ ID NO: 2.

In some embodiments of the present application, the Pembrolizumab variant comprises four peptide chains, wherein the first peptide chain of the variant comprises the amino acid sequence set forth in SEQ ID NO: 4, the second peptide chain comprises the amino acid sequence set forth in SEQ ID NO: 5, and the third peptide chain and the fourth peptide chain comprise the amino acid sequence set forth in SEQ ID NO: 2.

In some embodiments, the present application achieves at least one or more of the following advantageous technical effects:

1. The Pembrolizumab variant in the composition of the present application comprises a N-terminal truncation of a heavy chain. The variant exhibits affected thermostability as compared with Pembrolizumab without the N-terminal truncation of the heavy chain. The present application, however, demonstrates that there is no significant change in the overall binding activity and thermostability of the composition when the content of the variant in the composition is less than or equal to about 2% of the total amount of Pembrolizumab in the composition (based on the percentage of the content of the heavy chain containing the N-terminal truncation accounting for the total content of the heavy chain containing the N-terminal truncation and the untruncated heavy chain).

2. The present application demonstrates that if the content of the Pembrolizumab variant comprising the N-terminal truncation of the heavy chain is evaluated to be no more than about 2% of the total amount of Pembrolizumab in a sample during the preparation of Pembrolizumab (based on the percentage of the content of the heavy chain containing the N-terminal truncation accounting for the total content of the heavy chain containing the N-terminal truncation and the untruncated heavy chain), there is no need to perform operations of removing variants or impurities in general, which simplifies the production process and saves production costs to some extent.

It is to be understood that the features, characteristics, components, or steps described in a particular aspect, embodiment, or example of the present application may be applied to any other aspect, embodiment, or example described herein unless in conflict with them.

The above disclosure generally describes the present invention, which is further exemplified by the following examples. These examples are described merely to illustrate the invention and are not intended to limit the scope of the invention. Although specific terms and values are used herein, such terms and values are also to be understood as exemplary and do not limit the scope of the invention. Unless otherwise specified, the experimental methods and techniques in this specification are those well known to those skilled in the art.

## Examples

### Example 1: Preparation of Pembrolizumab

### Upstream cell culture process

In this example, an expression system, the CHOZN®GS expression system from Merck, was employed, which included a CHO-K1 host cell for cGMP library construction. The expression vector was pCGS3.2 plasmid from Merck that enabled efficient expression of exogenous recombinant proteins in mammal cells. The medium system included EX-CELL® CD CHO Fusion medium, and cell boost5 medium.

In accordance with typical cell culture processes for antibody drugs, the production of a stock solution of Pembrolizumab was carried out according to a process of cell recovery, step-by-step expansion in a shake flask and a bioreactor, and production in a 500 L disposable bioreactor, in which a feeding batch culture process was used in the production stage.

A cell cryopreservation tube was taken from a working cell bank, and thawed in a water bath at 37°C. The cells were cultured in a shake-flask expansion medium, successively expanded in a shake flask and a bioreactor, and then inoculated into a 500 L disposable bioreactor at a target density of $(4.5\pm1.0)\times10^6$ cells/ml.

The temperature was set to 36.5°C in the growth stage, and cooled down to 30°C in the expression stage. The pH was set to $7.0\pm0.3$, the dissolved oxygen was set to 40%, and the stirring speed was 50-70 rpm. Air was supplied from the top at a rate of 0-15 L/min, and from the bottom at a rate of 0-10 L/min. A pH control was coupled with bottom aeration (carbon dioxide) and supply of sodium carbonate solution, and a dissolved oxygen control was coupled with bottom aeration (oxygen). During the culture process, a feed medium was added in a volume which was 28-48% of the initial volume. A glucose solution was added as needed for cell growth, and an antifoaming agent solution was supplemented as needed for culture. The culture period was 16 days. The cell culture solution was subjected to deep filtration and sterilization with 0.2 μm filtration. The supernatant was collected for purification.

## Downstream purification process

In this example, typical purification processes for antibody drugs were employed, including affinity chromatography, low pH viral inactivation, anion exchange chromatography, cation exchange chromatography, virus removal filtration, ultrafiltration concentration, and finally addition of excipients and filtration to obtain a stock solution.

Protein A affinity chromatography. Protein A affinity chromatography was used as the primary capture step, using AT Protein A Diamond plus fillers from the manufacturer Bestchrom. The target antibody was bound to the fillers, while impurities of culture medium components and host cell proteins were not bound. After equilibrating the chromatography column with a Tris solution, a loading phase was started. At the end of the loading, the chromatography column was rinsed, and a solution of the eluted protein was collected.

Low pH viral inactivation. The eluate solution from the protein A affinity chromatography was adjusted to a low pH with acetic acid, to inactivate viruses that may be present. The product after the viral inactivation was neutralized with a Tris solution and filtered through a deep filter, to obtain a sample from the viral inactivation and neutralization filtration, which was further processed in a subsequent step of anion exchange chromatography.

Anion exchange chromatography. Toyopearl® NH2-750F fillers from TOSOH were used to perform the anion chromatography. They were equilibrated with a Tris buffer, and loaded with the product from the viral inactivation and neutralization filtration. The target protein flowed through the chromatography column during the loading. During the loading and the equilibrium process followed the loading, the target protein was collected according to a collection interval, to obtain a solution collected from the anion exchange chromatography.

Cation exchange chromatography. The cation chromatography was carried out with Capto SP ImpRes fillers from Cytiva, and the cation exchange chromatography column was equilibrated with a sodium acetate-acetic acid buffer. The protein solution collected from the anion exchange chromatography was loaded, allowing the protein to bind onto the cation exchange chromatography fillers. At the end of the loading, the target protein was eluted from the cation exchange chromatography column by gradient elution, to collect a solution of the eluted protein.

Virus removal filtration. The solution collected from the cation exchange chromatography was subjected to virus removal filtration with a virus removal prefilter (A1HC, as a cellulose-diatomaceous earth material) and a virus removal filter (Viresolve® Pro, as a PES material). At the end of the filtration, pipelines and filters were overwashed with a buffer, and the filtered sample was collected for filtration.

Ultrafiltration concentration: The antibody was adjusted to a desired concentration by ultrafiltration concentration. Upon completion of the ultrafiltration concentration, the excipient to be added was added to the antibody solution and finally the protein concentration was adjusted to about 165 mg/ml. Two formulations were prepared. Formulation 1 contained about 165 mg/ml Pembrolizumab, 10 mM histidine, 2% sucrose, 2% L-arginine, 15 mM methionine, 0.4 mg/ml polysorbate 80, pH 5.5, and 2000 IU/ml hyaluronidase. Formulation 2 contained about 165 mg/ml Pembrolizumab, 10 mM histidine, 7% sucrose, 15 mM methionine, 0.4 mg/ml polysorbate 80, pH 5.5, and 2000 IU/ml hyaluronidase.

## Example 2: Identification of N-terminal truncation of heavy chain of Pembrolizumab

A sample of Formulation 1 prepared in Example 1 was subjected to liquid chromatography-tandem high-resolution mass spectrometry with the following steps.

The sample was added with urea at a final concentration of 6 M and 10 mM TCEP and incubated at 37°C for 20 min for denaturation and reduction. After the incubation, iodoacetamide (IAM) was added at a final concentration of 20 mM. Incubation was performed for 15 min at 37°C in the dark for alkylation. The urea was then diluted to a concentration of 1 M by the addition of a solution of 50 mM ammonium bicarbonate. Sequencing-grade trypsin (Manufacturer: Rhinogen, Catalog No.: QIP-003A) was added at a ratio of protein: enzyme = 10:1 (mass ratio) and enzymatic cleavage was performed for 25 min at 37°C. At the end of the enzymatic cleavage, formic acid was added at a final concentration of 1% to stop the enzymatic cleavage. Centrifugation was performed at 12000 rpm for 3 min and the supernatant was transferred to a sample vial.

The sample to be tested was analyzed with liquid chromatography-tandem high-resolution mass spectrometry, with an injection volume of 10 μg. Targeted collection of information from first-order mass spectrometry and second-order mass spectrometry of the main ionic form (having a mass-to-charge ratio of 531.31) of a peptide segment in the truncation variant with the N-terminal truncation of the heavy chain (hereinafter referred to as the "truncated peptide segment", whose sequence is: GVEVK (SEQ ID NO:6), with the deleted amino acid residues marked with a strikethrough) was performed, using the following conditions for chromatography and mass spectrometry:

Conditions for chromatography:

| LC system | UHPLC, Vanquish horizon, Thermo |
|---|---|
| Chromatography column | ACQUITY Premier BEH C18 column, 1.7 μm particle size, 2.1×100 mm |
| Colum temperature | 60°C |
| Sample chamber temperature | 8°C |
| Mobile phase | A: a solution of 0.1% formic acid/water; B: a solution of 0.08% formic acid/80% acetonitrile/20% water |
| Elution gradient | After 1% phase B was used for equilibration for 3 min, phase B increased linearly to 45% in 57 min |

Conditions for mass spectrometry:

| Mass spectrometer | Orbitrap Exploris 480, Thermo |
|---|---|
| Ionization mode | H-ESI positive |
| Capillary voltage | 3.8 kV |
| Ion transfer tube temperature | 320°C |
| Vaporizer temperature | 300°C |
| Resolution of first-order mass spectrometry | 90000 |
| AGC Target of first-order mass spectrometry | 1e5 |
| Resolution of second-order mass spectrometry | 15000 |
| AGC Target of second-order mass spectrometry | 1e5 |
| HCD Collision Energy (NCE) | 27% |

Data processing was performed with Xcalibur software. The extracted ion current chromatogram (XIC) of the first-order mass spectrum, the first-order mass spectrum, and the second-order mass spectrum of the truncated peptide segment were shown in FIG. 1.

Results of the peptide mass fingerprinting indicated that the truncated peptide segment was identified at a retention time of 7.60 min in the sample. The measured m/z of the truncated peptide segment was 531.3144, while the theoretical m/z was 531.3137. The error was only 1.32 ppm. Meanwhile, fragment ions were able to be well matched with the theoretical b and y ions of the peptide segment in the second-order mass spectrum, thereby confirming the amino acid sequence of the truncated peptide segment.

**Example 3: Preparation of Pembrolizumab samples with different truncation ratios and determination of content thereof**

Recombinant expression of the Pembrolizumab variant with the N-terminal truncation was performed according to the identification results. If a wild-type Fc sequence was used for expression, a protein sample of three mixed components was expected to be found: a component with two truncated chains, a component with one truncated chain, and a component with two untruncated chains. To improve the successful pairing rate of heterodimers and to obtain relatively pure component with one truncated chain, a Knob-into-Hole (KiH) design was introduced for the recombinant expression of the Pembrolizumab variant with the N-terminal truncation, whose sequence information is shown in Table 1. Specifically, the two heavy chains designed in this example were heterologous, wherein the heavy chain with the N-terminal truncation was designed to have a "knob" structure comprising substitutions at two amino acid positions, S354C and T366W, while the heavy chain without the N-terminal truncation is designed to have a "hole" structure comprising substitutions at four amino acid positions, Y349C, T366S, L368A and Y407V. The heavy chain, in which the "hole" structure is located, further included H435R and Y436F substitutions for facilitating antibody purification.

Table 1. Sequences of Pembrolizumab variant S7 for Recombinant expression

| Chain | Sequence |
|---|---|
| Chain 1 | >K-H-knob<br><br>GVEVKKPGASVKVSCKASGYTFTNYYMYWVRQAPGQGLE WMGGINPSNGGTNFNEKFKNRVTLTTDSSTTTAYMELKSLQF DDTAVYYCARRDYRFDMGFDYWGQGTTVTVSSASTKGPSV FPLAPCSRSTSESTAALGCLVKDYFPEPVTVSWNSGALTSGV HTFPAVLQSSGLYSLSSVVTVPSSSLGTKTYTCNVDHKPSNTK VDKRVESKYGPPCPPCPAPEFLGGPSVFLFPPKPKDTLMISRTP EVTCVVVDVSQEDPEVQFNWYVDGVEVHNAKTKPREEQFN STYRVVSVLTVLHQDWLNGKEYKCKVSNKGLPSSIEKTISKA KGQPREPQVYTLPP*C*QEEMTKNQVSL*W*CLVKGFYPSDIAVE WESNGQPENNYKTTPPVLDSDGSFFLYSRLTVDKSRWQEGN VFSCSVMHEALHNHYTQKSLSLSLGK **(SEQ ID NO:5)** |
| Chain 2 | >K-H-hole<br><br>QVQLVQSGVEVKKPGASVKVSCKASGYTFTNYYMYWVRQA PGQGLEWMGGINPSNGGTNFNEKFKNRVTLTTDSSTTTAYM ELKSLQFDDTAVYYCARRDYRFDMGFDYWGQGTTVTVSSA STKGPSVFPLAPCSRSTSESTAALGCLVKDYFPEPVTVSWNSG ALTSGVHTFPAVLQSSGLYSLSSVVTVPSSSLGTKTYTCNVD HKPSNTKVDKRVESKYGPPCPPCPAPEFLGGPSVFLFPPKPKD TLMISRTPEVTCVVVDVSQEDPEVQFNWYVDGVEVHNAKTK PREEQFNSTYRVVSVLTVLHQDWLNGKEYKCKVSNKGLPSSI EKTISKAKGQPREPQV*C*TLPPSQEEMTKNQVSL*SCA*VKGFYPS DIAVEWESNGQPENNYKTTPPVLDSDGSFFL*V*SRLTVDKSRW QEGNVFSCSVMHEALHN*RF*TQKSLSLSLGK **(SEQ ID NO:4)** |
| Chain 3 | >K-L<br><br>EIVLTQSPATLSLSPGERATLSCRASKGVSTSGYSYLHWYQQ KPGQAPRLLIYLASYLESGVPARFSGSGSGTDFTLTISSLEPED |

(continued)

| Chain | Sequence |
|---|---|
|  | FAVYYCQHSRDLPLTFGGGTKVEIKRTVAAPSVFIFPPSDEQL KSGTASVVCLLNNFYPREAKVQWKVDNALQSGNSQESVTEQ DSKDSTYSLSSTLTLSKADYEKHKVYACEVTHQGLSSPVTKS FNRGEC **(SEQ ID NO:2)** |

| Note: The residues shown in italics are mutated amino acids. |
|---|

The plasmids were transfected into CHOZN cells by electroporation to obtain a stable expression cell pool after recovery. After 16 days of cell culture, the culture solution was harvested, filtered through a filter of 0.22 μm pore size and subjected to Protein A affinity chromatography. After ultrafiltration, diafiltration and concentration, the desired high-concentration Pembrolizumab variant S7 was obtained.

The S7 sample had a main peak of SEC purity at 90.6%. Results of complete molecular weight analysis showed that the observed molecular weight was consistent with the theoretical molecular weight.

The prepared S7 was added to samples of Formulation 2 prepared in Example 1 in a gradient, and the content of S7 in each sample was detected.

The contents of S7 in the samples were determined with peptide mass fingerprint analysis. Steps of the analysis were as follows.

The sampled were added with urea at a final concentration of 6 M and 10 mM TCEP and incubated at 37°C for 20 min for denaturation and reduction. IAM was added at a final concentration of 20 mM. Incubation was performed for 15 min at 37°C for alkylation. The urea was then diluted to a concentration of 1 M by the addition of a solution of 50 mM ammonium bicarbonate. Sequencing-grade trypsin (Manufacturer: Rhinogen, Catalog No.: QIP-003A) was added at a ratio of protein: enzyme = 10:1 (mass ratio) and enzymatic cleavage was performed for 25 min at 37°C. At the end of the enzymatic cleavage, formic acid was added at a final concentration of 1% to stop the enzymatic cleavage. Centrifugation was performed at 12000 rpm for 3 min and the supernatant was transferred to a sample vial.

The peptide mass fingerprinting analysis was performed with liquid chromatography-tandem high-resolution mass spectrometry, with an injection volume of 3 μg. Conditions for chromatography and mass spectrometry were as follows.

Conditions for chromatography:

| LC system | UHPLC, Vanquish Flex, Thermo |
|---|---|
| Chromatography column | ACQUITY Premier BEH C18 column, 1.7 μm particle size, 2.1×100 mm |
| Colum temperature | 60°C |
| Sample chamber temperature | 8°C |
| Mobile phase | A: a solution of 0.1% formic acid/water; B: a solution of 0.08% formic acid/80% acetonitrile/20% water |
| Elution gradient | After 1% phase B was used for equilibration for 3 min, phase B increased linearly to 45% in 57 min |

Conditions for mass spectrometry:

| MS system | Q-Exactive Plus, Thermo |
|---|---|
| Ionization mode | ESI positive |

(continued)

| Scan range of first-order mass spectrometry | 200-2000 m/z |
|---|---|
| Capillary voltage | 3.2 kV |
| Capillary Temp | 320°C |
| Resolution of first-order mass spectrometry | 70000 |
| AGC Target of first-order mass spectrometry | 1e6 |
| Resolution of second-order mass spectrometry | 17500 |
| AGC Target of second-order mass spectrometry | 1e5 |
| Selected ion window if second-order mass spectrometry | 1.6 m/z |

Data analysis was performed with Xcalibur software. XICs of two mostly responsive charge forms of the truncated peptide segment (GVEVK (SEQ ID NO:6); the mass-to-charge ratios of the major ions: 531.31 and 266.16) and its corresponding untruncated peptide segment (QVQLVQSGVEVK (SEQ ID NO:7); the mass-to-charge ratios of the major ions: 657.37 and 438.59 (N-terminal glutamine not cyclized), and 1296.74 and 648.87 (N-terminal glutamine cyclized)) were extracted. Peak areas of the respective target components were obtained through integration, and the truncation ratio was calculated according to the following formula:

$$\text{content of S7} = \frac{\text{XIC peak area of truncated peptide segment}}{\text{XIC peak area of truncated peptide segment} + \text{XIC peak area of untruncated peptide segment}} \times 100\%$$

The contents of S7 were shown in Table 2.

Table 2. The content of S7 in each sample

| Sample Name | S7 Content (%) |
|---|---|
| Addition Sample 1 | 34.49 |
| Addition Sample 2 | 22.80 |
| Addition Sample 3 | 12.94 |
| Addition Sample 4 | 4.15 |
| Addition Sample 5 | 1.99 |
| Addition Sample 6 | 1.51 |

**Example 4: Determination of binding activity of Pembrolizumab samples with different truncation ratios and DSC determination**

**Determination of binding activity of Pembrolizumab samples with different truncation ratios**

A study on the binding activity of the sample of Formulation 2 prepared in Example 1 and the samples prepared in Example 3 was performed.

The ability of the samples to compete with PD-L1 for binding to PD-1 was determined by time-resolved-fluorescence resonance energy transfer (TR-FRET) as follows.

Firstly, a control sample and test samples were subjected to gradient dilution with a diluent solution and added to a 96-well shallow-well plate at 5 μl/well. Then, diluted PD1 (His-tagged) protein and biotin-labeled PD-L1 protein were each added to the plate at 5 μl/well. 100-fold dilutions of streptavidin-d2 and MAb Anti-6HIS-Tb cryptate Gold were then mixed in equal volumes and added to the 96-well shallow-well plate at 10 μl/well. The plate was sealed with a sealing film and incubated at room temperature for 2 hours. The incubated 96-well shallow-well plate was briefly centrifuged at 1000 rpm and read using the time-resolved fluorescence function of a microplate reader (SPARK Multifunctional Microplate Reader from TECAN) at an excitation wavelength of 320 nm and emission wavelengths of 620 nm and 665 nm.

With sample concentrations as the X-axis, and TR-FRET ratios (signal values at 665 nm ÷ signal values at 620 nm × 10000, automatically calculated by the software after setting) as the Y-axis, a four-parameter fitting was performed to calculate the results.

The binding activities of Addition Sample 1, Addition Sample 2, Addition Sample 3, and Addition Sample 4 prepared in Example 3 were determined, with the Formulation 2 sample prepared in Example 1 as a control sample (the PD-1 binding activity of the control sample was set as 100%). Results of the activity determination are shown in Table 3 below. The analytic results showed that the binding activities of the various samples to PD-1 were from 80% to 120% of the control sample.

The binding activity of Pembrolizumab and its variant S7 to PD-1 in each sample was shown in Table 3.

Table 3. Binding activities to PD-1 of samples with different S7 contents

| Sample Name | Binding Activity (%) |
|---|---|
| Control Sample | 100 |
| Addition Sample 1 | 98 |
| Addition Sample 2 | 93 |
| Addition Sample 3 | 93 |
| Addition Sample 4 | 95 |

### DSC Determination of Pembrolizumab samples with different truncation ratios

DSC is a widely recognized analytical technique for characterizing thermostability of high-order structures in biological samples, allowing for real-time measurement and analysis of the heat capacity change of the samples in the process of controllable temperature rise, so as to obtain thermodynamic characterization parameters of the thermostability of the samples. It is a key technique for investigation and characterization of high-order structures in protein drugs. DSC analysis on the sample of Formulation 2 prepared in Example 1 and the samples prepared in Example 3 was performed with a Malvern Microcal TM VP-Capillary differential scanning calorimeter.

Parameter Settings:

| Parameter Category | Parameter Value |
|---|---|
| Scan initiation temperature | 20°C |
| Scan termination temperature | 100°C |
| Temperature scan rate | 90°C/hr |
| Signal feedback mode | None |
| Re-scanning opened or not | Not opened |
| Reagent for cleaning sample chambers | 14% (v/v) aqueous solution of Decon-90 |
| Reagent for cleaning at the end of testing | 14% (v/v) aqueous solution of Decon-90 |

The samples to be tested were respectively diluted to 1 mg/ml with a blank excipient buffer, and determination was performed, with the blank excipient buffer as a blank control. The measurements of Enthalpy Change ($\Delta H$) and Melting Temperature (Tm) of the samples with different S7 contents were shown in Table 4 and Table 5.

Table 4. $\Delta H$ and Tm Values of Samples with Different S7 Contents

| Sample Name | S7 Content (%) | $\Delta H$ (Cal/M) | Tm (°C) |
|---|---|---|---|
| Control Sample | < 0.01 | 550200 | 74.8 |
| Addition Sample 1 | 34.49 | 276400 | 74.7 |
| Addition Sample 2 | 22.80 | 352000 | 74.7 |
| Addition Sample 3 | 12.94 | 464500 | 74.7 |

(continued)

| Sample Name | S7 Content (%) | ΔH (Cal/M) | Tm (°C) |
|---|---|---|---|
| Addition Sample 4 | 4.15 | 497600 | 74.8 |

Note: The data in Table 4 is that from a first head-to-head experiment.

Table 5. ΔH and Tm Values of Samples of Different S7 Contents

| Sample Name | S7 Content (%) | ΔH (Cal/M) | Tm (°C) |
|---|---|---|---|
| Control Sample | < 0.01 | 596800 | 74.8 |
| Addition Sample 5 | 1.99 | 606800 | 74.8 |
| Addition Sample 6 | 1.51 | 593900 | 74.8 |

Note: The data in Table 5 is that from a second head-to-head experiment.

The results showed that the Tm values (Melting Temperature) of the samples of the truncation variant at different contents were not significantly different from that of the control sample, indicating that the types (including hydrophobic cores, disulfide bonds, etc.) and strengths of key intermolecular forces of the different samples were consistent. However, as the content of S7 gradually increased, the enthalpy change (ΔH) of the samples gradually decreased, that is, as the content of S7 gradually increased, the heat absorption during the protein denaturation process decreased, indicating that the thermostability of the truncation variant became worse than that of the normal sample. When the content of S7 was about 1.99%, the enthalpy change in heat of the samples was not significantly different from that of the control sample.

Various modifications and equivalents may be made to the embodiments disclosed in the present application without departing from the spirit and scope of the disclosure. Unless otherwise specified in the context, any features, steps or embodiments of the embodiments of the present disclosure may be used in combination with any other features or embodiments.

## Claims

1. A composition, wherein the composition comprises at least 100 mg/ml, at least 110 mg/ml, at least 120 mg/ml, at least 130 mg/ml, at least 140 mg/ml, at least 150 mg/ml, at least 160 mg/ml, at least 170 mg/ml, at least 180 mg/ml, at least 190 mg/ml, or at least 200 mg/ml of Pembrolizumab and a variant thereof;

   wherein the variant is a truncation variant with a N-terminal truncation of a heavy chain of Pembrolizumab, the variant comprising four peptide chains, wherein a first peptide chain of the variant comprises the amino acid sequence set forth in SEQ ID NO: 1 or SEQ ID NO: 3, a second peptide chain comprises the amino acid sequence set forth in SEQ ID NO: 3, and a third peptide chain and a fourth peptide chain comprise the amino acid sequence set forth in SEQ ID NO: 2; and
   wherein the content of the heavy chain comprising the N-terminal truncation is less than or equal to about 2%, expressed as a percentage of the content of the heavy chain comprising the N-terminal truncation accounting for the total content of heavy chains of Pembrolizumab and the variant thereof in the composition, which is calculated based on a trypsin-cleaved peptide mass fingerprinting analysis.

2. The composition of claim 1, wherein the first peptide chain of the variant comprises the amino acid sequence set forth in SEQ ID NO: 1, the second peptide chain comprises the amino acid sequence set forth in SEQ ID NO: 3, and the third peptide chain and the fourth peptide chain comprise the amino acid sequence set forth in SEQ ID NO: 2.

3. The composition of claim 1 or 2, wherein the content of the heavy chain comprising the N-terminal truncation is less than or equal to about 2.0%, less than or equal to about 1.9%, less than or equal to about 1.8%, less than or equal to about 1.7%, less than or equal to about 1.6%, less than or equal to about 1.5%, less than or equal to about 1.4%, less than or equal to about 1.3%, less than or equal to about 1.2%, less than or equal to about 1.1%, less than or equal to about 1.0%, less than or equal to about 0.9%, less than or equal to about 0.8%, less than or equal to about 0.7%, less than or equal to about 0.6%, less than or equal to about 0.5%, less than or equal to about 0.4%, less than or equal to about 0.3%, less than or equal to about 0.2%, or less than or equal to about 0.1%, expressed as a percentage of the content of the heavy chain comprising the N-terminal truncation accounting for the total content of heavy chains of Pembrolizumab and the variant thereof in the composition.

4. A pharmaceutical formulation comprising the composition of any one of claims 1 to 3, and one or more pharmaceutically acceptable carriers.

5. A method of detecting a Pembrolizumab variant in a Pembrolizumab-containing composition or pharmaceutical formulation, wherein the variant is a truncation variant with a N-terminal truncation of a heavy chain of Pembrolizumab, the variant comprising four peptide chains, wherein a first peptide chain of the variant comprises the amino acid sequence set forth in SEQ ID NO: 1 or SEQ ID NO: 3, a second peptide chain comprises the amino acid sequence set forth in SEQ ID NO: 3, and a third peptide chain and a fourth peptide chain comprise the amino acid sequence set forth in SEQ ID NO: 2, the method comprising:

   subjecting the composition or pharmaceutical formulation to enzymatic cleavage with trypsin to obtain an enzymatically cleaved product; and
   subjecting the enzymatically cleaved product to a peptide mass fingerprinting analysis, and determining the presence or absence of the Pembrolizumab variant based on the first-order and second-order mass spectra; wherein the composition or pharmaceutical formulation comprises at least 100 mg/ml, at least 110 mg/ml, at least 120 mg/ml, at least 130 mg/ml, at least 140 mg/ml, at least 150 mg/ml, at least 160 mg/ml, at least 170 mg/ml, at least 180 mg/ml, at least 190 mg/ml, or at least 200 mg/ml of Pembrolizumab and the variant thereof.

6. The method of claim 5, wherein the peptide mass fingerprinting analysis is a peptide mass fingerprinting analysis using LC-MS/MS.

7. The method of claim 5 or 6, further comprising obtaining, after the peptide mass fingerprint analysis, extracted ion current chromatograms (XICs) of a peptide segment comprising the N-terminal truncation of the heavy chain and an untruncated peptide segment, and calculating the content of the Pembrolizumab variant comprising the N-terminal truncation of the heavy chain based on corresponding peak areas.

8. The method of claim 7, wherein the amino acid sequence of the peptide segment comprising the N-terminal truncation of the heavy chain is set forth in SEQ ID NO:6, and the amino acid sequence of the untruncated peptide segment is set forth in SEQ ID NO:7.

Figure 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202511307916